# EUROPÄISCHE PATENTSCHRIFT

(11) **EP 2 944 642 B1**
(45) Veröffentlichungstag und Bekanntmachung des Hinweises auf die Patenterteilung: **10.05.2017**
(21) Anmeldenummer: 15161605.9
(22) Anmeldetag: 30.03.2015
(51) Int. Cl.: C07F 7/18

(54) **VERFAHREN ZUR HERSTELLUNG VON HARNSTOFFHALTIGEN SILANEN**
PROCESS FOR PREPARING UREA-CONTAINING SILANES
PROCÉDÉ DE FABRICATION DE SILANES CONTENANT DE L'URÉE

(30) Priorität: 15.05.2014 DE 102014209215
(43) Veröffentlichungstag der Anmeldung: 18.11.2015
(73) Patentinhaber: Evonik Degussa GmbH, 45128 Essen (DE)
(72) Erfinder: Rosenstingl, Sebastian, 79618 Rheinfelden (DE); Moser, Ralph, 79104 Freiburg i. Br. (DE); Röben, Caren, 50670 Köln (DE); Burger, Rosemarie, 79594 Inzlingen (DE)

(56) Entgegenhaltungen:
- JP-A- 2002 201 312
- ZHIGANG XU ET AL: "A new strategy to prepare glutathione responsive silica nanoparticles", RSC ADVANCES, Bd. 3, Nr. 39, 1. Januar 2013 (2013-01-01) , Seite 17700, XP055215911, ISSN: 2046-2069, DOI: 10.1039/c3ra43098g -& ZHIGANG XU ET AL: "Electronic Supplementary Information - A new strategy to prepare glutathione responsive silica nanoparticles", Royal Society of Chemistry , 7. August 2013 (2013-08-07), XP002744997, Gefunden im Internet: URL:http://www.rsc.org/suppdata/ra/c3/c3ra 43098g/c3ra43098g.pdf [gefunden am 2015-09-24]
- ERIC BESSON ET AL: "Soft route for monodisperse gold nanoparticles confined within SH-functionalized walls of mesoporous silica", JOURNAL OF MATERIALS CHEMISTRY, Bd. 19, Nr. 27, 1. Januar 2009 (2009-01-01), Seite 4746, XP055213535, ISSN: 0959-9428, DOI: 10.1039/b902568e
- MADHAV MANE ET AL: "An efficient and greener protocol towards synthesis of unsymmetrical N,N'-biphenyl urea", ARABIAN JOURNAL OF CHEMISTRY, Bd. 6, Nr. 4, 3. Februar 2011 (2011-02-03) , Seiten 423-427, XP055216003, ISSN: 1878-5352, DOI: 10.1016/j.arabjc.2011.01.030

## Beschreibung

Die Erfindung betrifft ein Verfahren zur Herstellung von harnstoffhaltigen Silanen.

Aus CAS 1184961-62-3, 442527-46-0 und 498553-03-0 sind Verbindungen der Formel 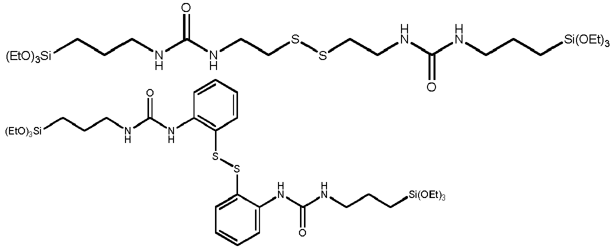 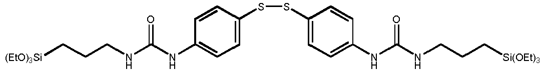 bekannt.

Ferner sind aus US 20030191270 A1 Silane der Formel 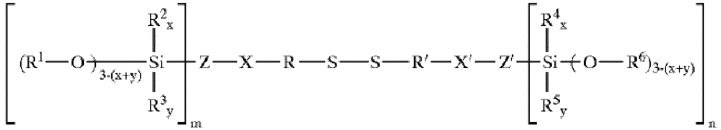 bekannt.

Aus JP 2002201312 A sind Kautschukmodifizierungsmittel der Formel 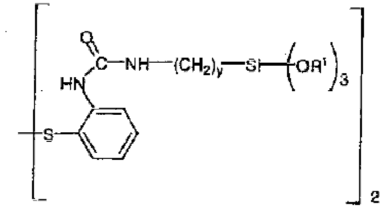 bekannt.

Ferner sind aus J. Mat. Chem. 2009, 19, 4746-4752 Goldnanopartikel innerhalb SHfunktionallisierter Gerüststrukturen aus mesoporösen Kieselsäuren und die Herstellung von harnstoffhaltigen Silanen bekannt. Bei dem bekannten Verfahren werden organische Lösungsmittel eingesetzt.

Nachteil des bekannten Herstellverfahrens ist, dass es sich um ein aufwendiges Verfahren (Neutralisation in H₂O, Extraktion in CH₂Cl₂-Phase, Trocknung mit MgSO₄, Lösungsmittelentfernung, Lösungsmittelwechsel auf THF, Reaktion in THF, Lösungsmittelentfernung, Fällung/Waschung in Pentan) mit vielen Verfahrensschritten und organischem Lösungsmittel handelt.

Aufgabe der vorliegenden Erfindung ist es, ein Verfahren zur Verfügung zu stellen, welches gegenüber den Verfahren aus dem Stand der Technik ohne organisches Lösungsmittel auskommt und weniger Verfahrensschritte aufweist.

Gegenstand der Erfindung ist ein Verfahren zur Herstellung von harnstoffhaltigen Silanen der allgemeinen Formel I 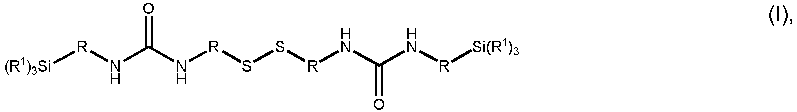 wobei R¹ gleich oder verschieden sind und C1-C10-Alkoxygruppen, vorzugsweise Methoxy- oder Ethoxygruppe, C2-C10- cyklische Dialkoxy-Gruppe, Phenoxygruppe, C4-C10-Cycloalkoxygruppen, C6-C20-Arylgruppen, vorzugsweise Phenyl, C1-C10-Alkylgruppe, vorzugsweise Methyl oder Ethyl, C2-C20-Alkenylgruppe, C7-C20-Aralkylgruppe oder Halogen, vorzugsweise Cl, und R gleich oder verschieden sind und eine verzweigte oder unverzweigte, gesättigte oder ungesättigte, aliphatische, aromatische oder gemischt aliphatische /aromatische zweibindige C1-C30-, bevorzugt C1-C20 -, besonders bevorzugt C1-C10-, ganz besonders bevorzugt C1-C7 -, insbesondere bevorzugt C2 und C3-,Kohlenwasserstoffgruppe, die gegebenenfalls mit F-, Cl-, Br-, I-, -CN oder HSsubstituiert ist, sind, welches dadurch gekennzeichnet ist,
dass man ein Diamin der allgemeinen Formel II

H₂N-R-S-S-R-NH₂ (II)

mit Isocyanatsilan der allgemeinen Formel III

(R¹)₃Si-R-NCO (III),

wobei R und R¹ die oben genannte Bedeutungen haben, in Wasser umsetzt, wobei die Umsetzung ohne organische Lösungsmittel durchgeführt wird.

Harnstoffhaltige Silane können Mischungen von harnstoffhaltigen Silanen der allgemeinen Formel I sein.

Das Verfahrensprodukt kann Oligomere, die durch Hydrolyse und Kondensation der Alkoxysilanfunktionen der harnstoffhaltigen Silanen der allgemeinen Formel I entstehen, enthalten.

R kann bevorzugt -CH₂-, -CH₂CH₂-, -CH₂CH₂CH₂-, -CH₂CH₂CH₂CH₂-, -CH(CH₃)-, -CH₂CH(CH₃)-, - CH(CH₃)CH₂-, -C(CH₃)₂-, -CH(C₂H₅)-, -CH₂CH₂CH(CH₃)-, -CH(CH₃)CH₂CH₂-, -CH₂CH(CH₃)CH₂-, -CH₂CH₂CH₂CH₂CH₂-, -CH₂CH₂CH₂CH₂CH₂CH₂-, -CH₂CH₂CH₂CH₂CH₂CH₂CH₂-, -CH₂CH₂CH₂CH₂CH₂CH₂CH₂CH₂-, -CH₂CH₂CH₂CH₂CH₂CH₂CH₂CH₂CH₂-, -CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂CH₂CH₂CH₂CH₂-
oder 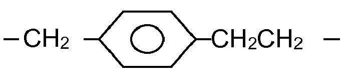 bzw. -CH₂-CH₂-C₆H₄-CH₂-bedeuten.

Harnstoffhaltige Silane der allgemeinen Formel I können bevorzugt sein:

((EtO)₃Si-CH₂-NH-CO-N-CH₂-S)₂,

((EtO)₃Si-CH₂CH₂-NH-CO-NH-CH₂-S)₂,

((EtO)₃Si-CH₂-NH-CO-NH-CH₂CH₂-S)₂,

((EtO)₃Si-CH₂CH2-NH-CO-NH-CH₂CH₂-S)₂,

((EtO)₃Si-CH₂CH₂CH₂-NH-CO-NH-CH₂-S)₂,

((EtO)₃Si-CH₂CH₂CH₂-NH-CO-NH-CH₂CH₂-S)₂,

((EtO)₃Si-CH₂-NH-CO-NH-CH₂CH₂CH₂-S)₂,

((EtO)₃Si-CH₂CH₂-NH-CO-NH-CH₂CH₂CH₂-S)₂,

((EtO)₃Si-CH₂CH₂CH₂-NH-CO-NH-CH₂CH₂CH₂-S)₂,

((MeO)₃Si-CH₂-NH-CO-NH-CH₂-S)₂,

((MeO)₃Si-CH₂CH₂-NH-CO-NH-CH₂-S)₂,

((MeO)₃Si-CH₂-NH-CO-NH-CH₂CH₂-S)₂,

((MeO)₃Si-CH₂CH₂-NH-CO-NH-CH₂CH₂-S)₂,

((MeO)₃Si-CH₂CH₂CH₂-NH-CO-NH-CH₂-S)₂,

((MeO)₃Si-CH₂CH₂CH₂-NH-CO-NH-CH₂CH₂-S)₂,

((MeO)₃Si-CH₂-NH-CO-NH-CH₂CH₂CH₂-S)₂,

((MeO)₃Si-CH₂CH₂-NH-CO-NH-CH₂CH₂CH₂-S)₂

oder

((MeO)₃Si-CH₂CH₂CH₂-NH-CO-NH-CH₂CH₂CH₂-S)₂.

Insbesondere bevorzugt ist die Verbindung der Formel

((EtO)₃Si-CH₂CH₂CH₂-NH-CO-NH-CH₂CH₂-S)₂.

Diamine der allgemeinen Formel II können bevorzugt sein:

H₂N-CH₂-S-S-CH₂-NH₂,

H₂N-CH₂CH₂-S-S-CH₂-NH₂,

H₂N-CH₂CH₂-S-S-CH₂CH₂-NH₂,

H₂N-CH₂-S-S-CH₂CH₂CH₂-NH₂,

H₂N-CH₂CH₂-S-S-CH₂CH₂CH₂-NH₂

oder

H₂N-CH₂CH₂CH₂-S-S-CH₂CH₂CH₂-NH₂.

Isocyanatsilane der allgemeinen Formel III können bevorzugt sein:

(C₂H₅O)₃Si-CH₂-NCO,

(C₂H₅O)₃Si-CH₂CH₂-NCO,

(C₂H₅O)₃Si-CH₂CH₂CH₂-NCO,

(CH₃O)₃Si-CH₂-NCO,

(CH₃O)₃Si-CH₂CH₂-NCO oder

(CH₃O)₃Si-CH₂CH₂CH₂-NCO.

Das durch das erfindungsgemäße Verfahren erhältliche harnstoffhaltige Silan der allgemeinen Formel I kann in einer Ausbeute von größer als 50%, bevorzugt größer als 60%, besonders bevorzugt größer als 70%, ganz besonders bevorzugt größer als 80%, erhalten werden.

Der lösliche Anteil in dem durch das erfindungsgemäße Verfahren erhaltenen Produkt in DMSO-d⁶ oder CDCl₃ wird durch Zugabe eines internen Standards, wie beispielsweise Triphenylphosphinoxid (TPPO), in DMSO-d6 oder in CDCl₃ und eine dem Fachmann bekannte ¹H-NMR Methode ermittelt.

Im Verhältnis zu den eingesetzten Diaminen der allgemeinen Formel II kann die Wassermenge mehr als 1 Gew.-%, bevorzugt mehr als 10 Gew.-%, besonders bevorzugt mehr als 50 Gew.-% und ganz besonders bevorzugt mehr als 100 Gew.-%, betragen.

Die Reaktion kann unter Luftausschluß durchgeführt werden.

Die Reaktion kann unter Schutzgasatmosphäre durchgeführt werden, zum Beispiel unter Argon oder Stickstoff, bevorzugt unter Stickstoff.

Das erfindungsgemäße Verfahren kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein.

Das erfindungsgemäße Verfahren kann zwischen 0°C und +100°C, bevorzugt zwischen 5°C und 60°C, besonders bevorzugt zwischen 5°C und 30°C durchgeführt werden.

Bei dem erfindungsgemäßen Verfahren können Diamine der allgemeinen Formel II zu Isocyanatsilanen der allgemeinen Formel II dosiert werden.

Bei dem erfindungsgemäßen Verfahren können bevorzugt die Isocyanatsilane der allgemeinen Formel III zu Diaminen der allgemeinen Formel II dosiert werden.

Die Diamine der allgemeinen Formel II können vor der Umsetzung mit den Isocyanatsilanen der allgemeinen Formel III aus den Hydrohalogenid-Salzen der Diamine der allgemeinen Formel IV

Hal^{- +}H₃N-R-S-S-R-NH₃⁺Hal⁻ (IV)

durch Zugabe einer Base, vorzugsweise NaOH oder KOH, hergestellt werden, wobei Hal, F, Cl, Br oder I, vorzugsweise Cl, ist. Dabei kann die Base zugegeben werden bis sich ein pH-Wert zwischen 7 und 14 einstellt.

Bei dem erfindungsgemäßen Verfahren können die Diamine der allgemeinen Formel II zu Isocyantsilanen der allgemeinen Formel III im molekularen Verhältnis von 1:1,70 bis 1:2,20, bevorzugt 1:1,75 bis 1:2,10, besonders bevorzugt im Verhältnis 1:1,80 bis 1:2,00, eingesetzt werden.

Das Produkt, hergestellt mit dem erfindungsgemäßen Verfahren, kann einen Restgehalt an Diaminen der allgemeinen Formel II von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der Diamine der allgemeinen Formel II in dem Produkt, hergestellt mit dem erfindungsgemäßen Verfahren, werden im ¹³C-NMR durch Integration der C-Atome der -CH₂-NH₂ Gruppe der Diamine der allgemeinen Formel II gegen die C-Atome der Si-CH₂- Gruppe der harnstoffhaltigen Silane der allgemeinen Formel I bestimmt.
Für die Substanz der Formel II H₂N-CH₂-CH₂-S-S-CH₂-CH₂-NH₂ wird zum Beispiel das Integral der C-Atome der -CH₂-NH₂ Gruppe für die Bestimmung der relativen Gehalte verwendet.

Das Produkt, hergestellt mit dem erfindungsgemäßen Verfahren, kann einen Restgehalt an Isocyanatsilanen der allgemeinen Formel III von weniger als 10 mol-%, bevorzugt weniger als 5 mol-%, besonders bevorzugt weniger als 1 mol-%, ganz besonders bevorzugt weniger als 0,1 mol-%, haben.

Die relativen mol-% der Isocyanatsilane der allgemeinen Formel III in dem Produkt in einem Bereich >1 mol-%, hergestellt mit dem erfindungsgemäßen Verfahren, werden im ¹³C-NMR durch Integration der C-Atome der -NCO Gruppe der Isocyanatsilane der allgemeinen Formel III gegen die C-Atome der Si-CH₂- Gruppe der harnstoffhaltigen Silane der allgemeinen Formel I bestimmt. Für die Substanz der Formel III (EtO)₃Si-CH₂-CH₂-CH₂-NCO wird zum Beispiel das Integral der C-Atome der -NCO Gruppe (δ = 122,22 ppm) für die Bestimmung der relativen Gehalte in einem Bereich >1 mol-% verwendet.
Die relativen mol-% der Isocyanatsilane der allgemeinen Formel III in dem Produkt in einem Bereich <1 mol-%, hergestellt mit dem erfindungsgemäßen Verfahren, werden durch eine dem Fachmann bekannte quantitative FT-IR-Spektroskopie bestimmt. Die Kalibrierung der Methode erfolgt durch Verwendung von Kalibrierlösungen geeigneter Konzentration (z.B. in C₂Cl₄). Für die Messung werden ca. 1 g Probe in eine 25 ml Rollkragenflasche eingewogen und 25 g C₂Cl₄ zugegeben. Die Probe wird auf der Schüttelmaschine 1 - 2 Stunden geschüttelt. Im Anschluss wird die untere flüssige Phase vorsichtig in eine 20 mm IR-Küvette dosiert und per FT-IR-Spektroskopie vermessen (4000 -1200 cm-1, Auflösung 2 cm⁻¹). Unter gleichen Bedingungen wird ein Spektrum des Lösungsmittels zur Subtraktion aufgenommen.
Für die Substanz der Formel III (EtO)₃Si-CH₂-CH₂-CH₂-NCO wird zum Beispiel die Wellenlänge der Valenzschwingung der -NCO Gruppe bei 2270 cm⁻¹ für die Bestimmung der relativen Gehalte in einem Bereich <1 mol-% verwendet.

Das Produkt, hergestellt mit dem erfindungsgemäßen Verfahren, kann einen Restgehalt an Hydrohalogenid-Salz der Diamine der allgemeinen Formel IV von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der Hydrohalogenid-Salze der Diamine der allgemeinen Formel IV in dem Produkt, hergestellt mit dem erfindungsgemäßen Verfahren, werden im ¹³C-NMR durch Integration der C-Atome der -C-H₂-NH₂·HCl Gruppe der Hydrohalogenid-Salze der Diamine der allgemeinen Formel IV gegen die C-Atome der Si-CH₂- Gruppe der harnstoffhaltigen Silane der allgemeinen Formel I bestimmt.
Für die Substanz der Formel IV HCl·H₂N-CH₂-CH₂-S-S-CH₂-CH₂-NH₂·HCl wird zum Beispiel das Integral der C-Atome der S-CH₂-CH₂-NH₂ HCl Gruppe (δ = 37,82 ppm) oder der S-CH₂-CH₂-NH₂·HCl Gruppe (δ = 33,79 ppm) für die Bestimmung der relativen Gehalte verwendet.

Das Reaktionsprodukt kann anschließend gefiltert werden und mit Wasser und/oder einem organischen Lösungsmittel, vorzugsweise Alkan, besonders bevorzugt Pentan, Hexan oder Heptan, gewaschen werden. Bevorzugt kann mit Wasser und anschließend mit einem Alkan, besonders bevorzugt Hexan, gewaschen werden.

Das Produkt kann nach der Filterung getrocknet werden. Die Trocknung kann bei Temperaturen von 20°C- 100°C, vorzugsweise von 25°C- 50°C, erfolgen. Die Trocknung kann bei Unterdruck von 1 - 500 mbar erfolgen.

In einer bevorzugten Ausführungsform kann das Verfahren zur Herstellung von harnstoffhaltigen Silanen der allgemeinen Formel I 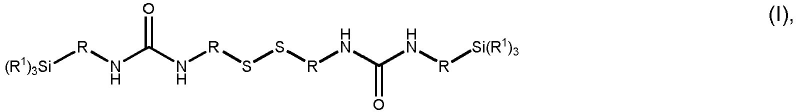 wobei R und R¹ die oben genannte Bedeutung haben, dadurch gekennzeichnet sein, dass man das Hydrohalogenidsalz des
Diamins der allgemeinen Formel IV

Hal^{- +}H₃N-R-S-S-R-NH₃⁺Hal⁻ (IV)

in Wasser löst und mit einer Base umsetzt,
anschließend das Isocyanatsilan der allgemeinen Formel III

(R¹)₃Si-R-NCO (III),

zugibt, das ausgefallene Produkt abfiltriert, mit Wasser und Hexan wäscht und trocknet.

Die harnstoffhaltigen Silane der allgemeinen Formel I können als Haftvermittler zwischen anorganischen Materialien, zum Beispiel
Glaskugeln, Glasplittern, Glasoberflächen, Glasfasern, oder oxidischen Füllstoffen, bevorzugt Kieselsäuren wie gefällten Kieselsäuren und pyrogene Kieselsäuren,
und organischen Polymeren, zum Beispiel Duroplasten, Thermoplasten oder Elastomeren, beziehungsweise als Vernetzungsmittel und Oberflächenmodifizierungsmittel für oxidische Oberflächen verwendet werden.

Die harnstoffhaltigen Silane der allgemeinen Formel I können als Kopplungsreagenzien in gefüllten Kautschukmischungen, beispielsweise Reifenlaufflächen, technischen Gummiartikeln oder Schuhsolen, verwendet werden.

Vorteil des erfindungsgemäßen Verfahrens ist, dass die Herstellung von harnstoffhaltigen Silanen der allgemeinen Formel I in einem Syntheseschritt ohne organisches Lösungsmittel möglich ist.

Ein weiterer Vorteil des erfindungsgemäßen Verfahrens ist, dass die Umsetzung in relativ kurzer Zeit erfolgt.

Ein weiterer Vorteil des erfindungsgemäßen Verfahrens ist, dass auf eine aufwendige Aufreinigung der erhaltenen Produkte verzichtet werden kann.

### Beispiele:

### Beispiel 1: Herstellung von [(EtO)₃Si-(CH₂)₃-NH-C(=O)-NH-(CH₂)₂-S-]₂ in Wasser (mit Hexan-Waschung)

In einem mit N2 gespülten 2L Doppelmantel-Vierhalskolben mit KPG-Rührer, Rückflusskühler, Innenthermometer und Tropftrichter wird Cystamindihydrochlorid (108,39 g, 0,47 mol, 1.00 eq) vorgelegt und in VE-Wasser (940 mL) gelöst. Mittels Tropftrichter wird 50%ige KOH-Lösung (92,31 g, 0,82 mol, 1,75 eq) bei 17-20°C zudosiert und für 15 min gerührt. Nun wird 3-Isocyanatopropyltriethoxysilan (221,05 g, 0,85 mol, 1,8 eq) so zudosiert, dass eine Innentemperatur von 30°C nicht überschritten wird. Danach wird eine Stunde bei 23°C gerührt. Die weiße Suspension wird über Druck filtriert, mit 200 ml VE-Wasser gespült und mit trockenem N₂ für 2 h getrocknet. Der Filterkuchen wird mit drei Portionen Hexan (je 150 mL) gewaschen und erneut mit trockenem N₂ für 1 h getrocknet. Das Produkt [(EtO)₃Si-(CH₂)₃-NH-C(=O)-NH-(CH₂)₂-S-]₂ ist ein feines, weißes Pulver (254,78 g, 92,8% d.Th.);
¹H-NMR (δₚₚₘ, 500 MHz, CDCl₃): 0.64 (4H, t), 1.22 (18H, t), 1.61 (4H, m), 2.78 (4H, m), 3.15 (4H, m), 3.52 (4H, m), 3.81 (12H, q), 5.2-6.5 (4H, br);
¹³C-NMR (δₚₚₘ, 125 MHz, CDCl₃): 7.7 (2C), 18.3 (6C), 23.8 (2C), 38.8 (2C), 38.9 (2C), 42.8 (2C), 58.3 (6C), 159.0 (2C).
²⁹Si-NMR (δₚₚₘ, 100 MHz, CDCl₃): -45.7 (97,4% Silan), -53,5 (2,6% M-Strukturen);
lösliche Anteile in CDCl₃ unter Verwendung von internem

| | |
|---|---|
| Standard TPPO: | 94,4%; |
| Wassergehalt (DIN 51777): | 0,4%; |
| Schmelzbeginn: | 106-110°C; |
| Restgehalt Isocyanat: | 0,04% |

### Beispiel 2: Herstellung von [(EtO)₃Si-(CH₂)₃-NH-C(=O)-NH-(CH₂)₂-S-]₂ in Wasser (ohne Hexan-Waschung)

In einem mit N2 gespülten 1L Doppelmantel-Vierhalskolben mit KPG-Rührer, Rückflusskühler, Innenthermometer und Tropftrichter wird Cystamindihydrochlorid (108,39 g, 0,47 mol, 1.00 eq) vorgelegt und in VE-Wasser (382 mL) gelöst. Mittels Tropftrichter wird 50%ige KOH-Lösung (92,31 g, 0,82 mol, 1,75 eq) bei 15-23°C zudosiert und für 30 min gerührt. Nun wird 3-Isocyanatopropyltriethoxysilan (221,05 g, 0,85 mol, 1,8 eq) so zudosiert, dass eine Innentemperatur von 30°C nicht überschritten wird. Danach wird eine Stunde bei 24°C gerührt. Die weiße Suspension wird über Druck filtriert, mit drei Portionen VE-Wasser (340 mL gesamt) gespült und mit trockenem N₂ für 2 h getrocknet. Der Filterkuchen wird im Rotationsverdampfer für 7 h bei 35°C und 166 mbar, für 10 h bei 35°C und 150 mbar und für 9 h bei 35°C und 100 mbar im N₂-Strom getrocknet. Das Produkt [(EtO)₃Si-(CH₂)₃-NH-C(=O)-NH-(CH₂)₂-S-]₂ ist ein feines, weißes Pulver (246,38 g, 90,7% d.Th.);
¹H-NMR (δₚₚₘ, 500 MHz, DMSO-d6): 0.52 (4H, t), 1.14 (18H, t), 1.42 (4H, m), 2.74 (4H, m), 2.96 (4H, m), 3.29 (4H, m), 3.74 (12H, q), 6.05 (4H, m);
¹³C-NMR (δₚₚₘ, 125 MHz, DMSO-d6): 7.3 (2C), 18.2 (6C), 23.5 (2C), 38.5 (2C), 39.6 (2C), 42.0 (2C), 57.7 (6C) 157.9 (2C).
²⁹Si-NMR (δₚₚₘ, 100 MHz, DMSO-d6): -45.3 (100% Silan);
lösliche Anteile in d6-DMSO unter Verwendung von internem

| | |
|---|---|
| Standard TPPO: | 86,0%; |
| Wassergehalt (DIN 51777): | 0,7%; |
| Schmelzbeginn: | 97°C; |
| Restgehalt Isocyanat: | 0,08% |

### Beispiel 3: (Vergleichsbeispiel)

### Herstellung von [(EtO)₃Si-(CH₂)₃-NH-C(=O)-NH-(CH₂)₂-S-]₂ in organischen Lösungsmittel Dichlormethan und THF

In einem trockenem und mit N2 gespülten Dreihalskolben mit Rührer, Rückflusskühler, Innenthermometer und Tropftrichter wird Cystamindihydrochlorid (22,52 g, 0,10 mol, 1.00 eq) vorgelegt und in Dichlormethan (100 mL) gelöst. Mittels Tropftrichter wird 50%ige KOH-Lösung (11,5 mL, 0,20 mol, 2,00 eq) zudosiert. Die entstandene Suspension wird mit 50 mL VE-Wasser in Lösung gebracht, zusätzlich 6,5 mL 50%ige KOH-Lösung hinzugegeben und für 2 h gerührt. Die beiden Phasen werden getrennt und die wässrige Phase mit Dichlormethan (3 x 50 mL) extrahiert. Die vereinten organischen Phasen werden über MgSO₄ getrocknet, filtriert und am Rotationsverdampfer vom Lösungsmittel befreit. Das erhaltene hellgelbe, zähflüssige Öl (Cystamin, 12,07 g) wird in THF (100 mL) aufgenommen, mittels Tropftrichter 3-Isocyanatopropyltriethoxysilan (49,47 g, 0,20 mol, 2,00 eq) zudosiert und über Nacht bei 23°C gerührt. Nach beendeter Reaktion wird das Lösungsmittel am Rotationsverdampfer entfernt. Der entstehende weiße Feststoff wird in frischem THF aufgenommen (84 mL) und unter Zugabe von *n*-Pentan (521 mL) bei 8°C ausgefällt. Die Suspension wird über Druck filtriert, der Filterkuchen mit *n*-Pentan gewaschen (3 x 100 mL) und mit trockenem N₂ getrocknet. Das Produkt [(EtO)₃Si-(CH₂)₃-NH-C(=O)-NH-(CH₂)₂-S-]₂ ist ein feines, weißes Pulver (50,42 g, 77,9% d.Th.);
²⁹Si-NMR (δₚₚₘ, 100 MHz, DMSO-d6): -40.5 (91,2% Silan), -48,2 (8,9% M-Strukturen).

## Patentansprüche

1. Verfahren zur Herstellung von harnstoffhaltigen Silanen der allgemeinen Formel I 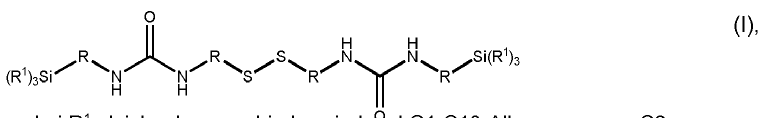 wobei R¹ gleich oder verschieden sind und C1-C10-Alkoxygruppen, C2-C10- cyklische Dialkoxy-Gruppe, Phenoxygruppe, C4-C10-Cycloalkoxygruppen, C6-C20-Arylgruppen, C1-C10-Alkylgruppe, C2-C20-Alkenylgruppe, C7-C20-Aralkylgruppe oder Halogen, und R gleich oder verschieden sind und eine verzweigte oder unverzweigte, gesättigte oder ungesättigte, aliphatische, aromatische oder gemischt aliphatische /aromatische zweibindige C₁-C₃₀-, Kohlenwasserstoffgruppe sind, **dadurch gekennzeichnet, dass** man
ein Diamin der allgemeinen Formel II
H₂N-R-S-S-R-NH₂ (II)
mit Isocyanatsilan der allgemeinen Formel III
(R¹)₃Si-R-NCO (III),
in Wasser umsetzt, wobei die Umsetzung ohne organisches Lösungsmittel durchgeführt wird.

2. Verfahren zur Herstellung von harnstoffhaltigen Silanen der allgemeinen Formel I gemäß Anspruch 1, **dadurch gekennzeichnet, dass** die harnstoffhaltigen Silane der allgemeinen Formel I
((EtO)₃Si-CH₂-NH-CO-NH-CH₂-S)₂,
((EtO)₃Si-CH₂CH₂-NH-CO-NH-CH₂-S)₂,
((EtO)₃Si-CH₂-NH-CO-NH-CH₂CH₂-S)₂,
((EtO)₃Si-CH₂CH₂-NH-CO-NH-CH₂CH₂-S)₂,
((EtO)₃Si-CH₂CH₂CH₂-NH-CO-NH-CH₂-S)₂,
((EtO)₃Si-CH₂CH₂CH₂-NH-CO-NH-CH₂CH₂-S)₂,
((EtO)₃Si-CH₂-NH-CO-NH-CH₂CH₂CH₂-S)₂,
((EtO)₃Si-CH₂CH₂-NH-CO-NH-CH₂CH₂CH₂-S)₂ oder
((EtO)₃Si-CH₂CH₂CH₂-NH-CO-NH-CH₂CH₂CH₂-S)₂,
sind.

3. Verfahren zur Herstellung von harnstoffhaltigen Silanen der allgemeinen Formel I gemäß Anspruch 1, **dadurch gekennzeichnet, dass** die Wassermenge größer 1 Gew.-% bezogen auf die eingesetzten Diamine der allgemeinen Formel II beträgt.

4. Verfahren zur Herstellung von harnstoffhaltigen Silanen der allgemeinen Formel I gemäß Anspruch 1, **dadurch gekennzeichnet, dass** die Temperatur zwischen 0°C und +100°C beträgt.

5. Verfahren zur Herstellung von harnstoffhaltigen Silanen der allgemeinen Formel I gemäß Anspruch 1, **dadurch gekennzeichnet, dass** die Diamine der allgemeinen Formel II vor der Umsetzung mit den Isocyanatsilanen der allgemeinen Formel III aus den Hydrohalogenid-Salzen der Diamine der allgemeinen Formel IV
Hal⁻⁺H₃N-R-S-S-R-NH₃⁺Hal⁻ (IV)
durch Zugabe einer Base hergestellt werden.

6. Verfahren zur Herstellung von harnstoffhaltigen Silanen der allgemeinen Formel I gemäß Anspruch 5, **dadurch gekennzeichnet, dass** man als Base NaOH oder KOH einsetzt.

7. Verfahren zur Herstellung von harnstoffhaltigen Silanen der allgemeinen Formel I gemäß Ansprüche 5 oder 6, **dadurch gekennzeichnet, dass** man die Base bis sich ein pH-Wert zwischen 7 und 14 einstellt zugibt.

8. Verfahren zur Herstellung von harnstoffhaltigen Silanen der allgemeinen Formel I gemäß Anspruch 1, **dadurch gekennzeichnet, dass** man Diamine der allgemeinen Formel II zu Isocyantsilanen der allgemeinen Formel III im molekularen Verhältnis von 1:1,80 bis 1:2,25 einsetzt.

9. Verfahren zur Herstellung von harnstoffhaltigen Silanen der allgemeinen Formel I gemäß Anspruch 1, **dadurch gekennzeichnet, dass** man das Reaktionsprodukt anschließend abfiltriert und mit Wasser und/oder organischem Lösungsmittel wäscht.

10. Verfahren zur Herstellung von harnstoffhaltigen Silanen der allgemeinen Formel I gemäß Anspruch 9, **dadurch gekennzeichnet, dass** man das abfiltrierte Produkt trocknet.

## Claims

1. Process for preparing urea-containing silanes of the general formula I 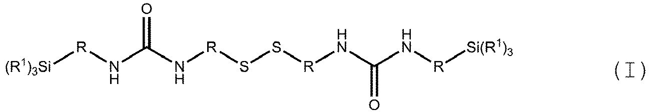 where R¹ are the same or different and are C1-C10 alkoxy groups, C2-C10 cyclic dialkoxy group, phenoxy group, C4-C10 cycloalkoxy groups, C6-C20 aryl groups, C1-C10 alkyl group, C2-C20 alkenyl group, C7-C20 aralkyl group or halogen, and R are the same or different and are a branched or unbranched, saturated or unsaturated, aliphatic, aromatic or mixed aliphatic/aromatic divalent C₁-C₃₀ hydrocarbon group, **characterized in that**
a diamine of the general formula II
H₂N-R-S-S-R-NH₂ (II)
is reacted with isocyanatosilane of the general formula III
(R¹)₃Si-R-NCO (III)
in water, wherein the reaction is conducted without organic solvent.

2. Process for preparing urea-containing silanes of the general formula I according to Claim 1, **characterized in that** the urea-containing silanes of the general formula I are
((EtO)₃Si-CH₂-NH-CO-NH-CH₂-S)₂,
((EtO)₃Si-CH₂CH₂-NH-CO-NH-CH₂-S)₂,
((EtO)₃Si-CH₂-NH-CO-NH-CH₂CH₂-S)₂,
((EtO)₃Si-CH₂CH₂-NH-CO-NH-CH₂CH₂-S)₂,
((EtO)₃Si-CH₂CH₂CH₂-NH-CO-NH-CH₂-S)₂,
((EtO)₃Si-CH₂CH₂CH₂-NH-CO-NH-CH₂CH₂-S)₂,
((EtO)₃Si-CH₂-NH-CO-NH-CH₂CH₂CH₂-S)₂,
((EtO)₃Si-CH₂CH₂-NH-CO-NH-CH₂CH₂CH₂-S)₂ or
((EtO)₃Si-CH₂CH₂CH₂-NH-CO-NH-CH₂CH₂CH₂-S)₂.

3. Process for preparing urea-containing silanes of the general formula I according to Claim 1, **characterized in that** the amount of water is greater than 1% by weight, based on the diamines of the general formula II used.

4. Process for preparing urea-containing silanes of the general formula I according to Claim 1, **characterized in that** the temperature is between 0°C and +100°C.

5. Process for preparing urea-containing silanes of the general formula I according to Claim 1, **characterized in that** the diamines of the general formula II, prior to reaction with the isocyanatosilanes of the general formula III, are prepared from the hydrohalide salts of the diamine of the general formula IV
Hal^{- +}H₃N-R-S-S-R-NH₃⁺Hal⁻ (IV)
by addition of a base.

6. Process for preparing urea-containing silanes of the general formula I according to Claim 5, **characterized in that** the base used is NaOH or KOH.

7. Process for preparing urea-containing silanes of the general formula I according to Claim 5 or 6, **characterized in that** the base is added until a pH between 7 and 14 is established.

8. Process for preparing urea-containing silanes of the general formula I according to Claim 1, **characterized in that** diamines of the general formula II are used relative to isocyanatosilanes of the general formula III in a molecular ratio of 1:1.80 to 1:2.25.

9. Process for preparing urea-containing silanes of the general formula I according to Claim 1, **characterized in that** the reaction product is subsequently filtered off and washed with water and/or organic solvent.

10. Process for preparing urea-containing silanes of the general formula I according to Claim 9, **characterized in that** the product which has been filtered off is dried.

## Revendications

1. Procédé pour la préparation de silanes contenant de l'urée, de formule générale I 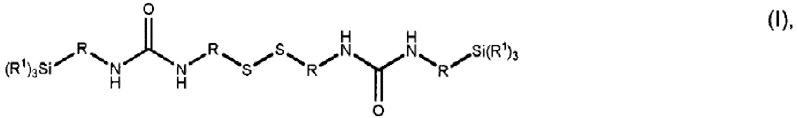 dans laquelle les radicaux R¹ sont identiques ou différents et représentent des groupes alcoxy en C₁-C₁₀, un groupe dialcoxy cyclique en C₂-C₁₀, un groupe phénoxy, des groupes cycloalcoxy en C₄-C₁₀, des groupes aryle en C₆-C₂₀, un groupe alkyle en C1-C₁₀, un groupe alcényle en C₂-C₂₀, un groupe aralkyle en C₇-C₂₀ ou des atomes d'halogène, et les radicaux R sont identiques ou différents et représentent un groupe hydrocarboné à deux liaisons, aliphatique, aromatique ou aliphatique/aromatique mixte en C₁-C₃₀, ramifié ou non ramifié, saturé ou insaturé, **caractérisé en ce qu'**on
fait réagir une diamine de formule générale II
H₂N-R-S-S-R-NH₂ (II)
avec un isocyanate-silane de formule générale III
(R¹)₃Si-R-NCO (III)
dans de l'eau, la réaction étant effectuée sans solvant organique.

2. Procédé pour la préparation de silanes contenant de l'urée, de formule générale I selon la revendication 1, **caractérisé en ce que** les silanes contenant de l'urée de formule générale I sont
((EtO)₃Si-CH₂-NH-CO-NH-CH₂-S)₂,
((EtO)₃Si-CH₂CH₂-NH-CO-NH-CH₂-S)₂,
((EtO)₃Si-CH₂-NH-CO-NH-CH₂CH₂-S)₂,
((EtO)₃Si-CH₂CH₂-NH-CO-NH-CH₂CH₂-S)2,
((Et-O)₃Si-CH₂CH₂CH₂-NH-CO-NH-CH₂-S)₂,
((Et-O)₃Si-CH₂CH₂CH₂-NH-CO-NH-CH₂CH₂-S)₂,
((EtO)₃Si-CH₂-NH-CO-NH-CH₂CH₂CH₂-S)₂,
((EtO)3Si-CH2CH₂-NH-CO-NH-CH₂CH₂CH₂-S)₂ ou
((EtO)₃Si-CH₂CH₂CH₂-NH-CO-NH-CH₂CH₂CH₂-S)₂.

3. Procédé pour la préparation de silanes contenant de l'urée, de formule générale I, selon la revendication 1, **caractérisé en ce que** la quantité d'eau est supérieure à 1 % en poids, par rapport aux diamines de formule générale II utilisées.

4. Procédé pour la préparation de silanes contenant de l'urée, de formule générale I, selon la revendication 1, **caractérisé en ce que** la température est comprise entre 0 °C et + 100 °C.

5. Procédé pour la préparation de silanes contenant de l'urée, de formule générale I, selon la revendication 1, **caractérisé en ce qu'**avant la réaction avec les isocyanate-silanes de formule générale III on prépare les diamines de formule générale II à partir des sels halogénohydrates des diamines de formule générale IV
Hal^{- +}H₃N-R-S-S-R-NH₃⁺ Hal⁻ (IV)
par addition d'une base.

6. Procédé pour la préparation de silanes contenant de l'urée, de formule générale I, selon la revendication 5, **caractérisé en ce qu'**on utilise comme base NaOH ou KOH.

7. Procédé pour la préparation de silanes contenant de l'urée, de formule générale I, selon la revendication 5 ou 6, **caractérisé en ce qu'**on ajoute la base jusqu'à ce que s'ajuste un pH entre 7 et 14.

8. Procédé pour la préparation de silanes contenant de l'urée, de formule générale I, selon la revendication 1, **caractérisé en ce qu'**on utilise des diamines de formule générale II en un rapport moléculaire avec les isocyanate-silanes de formule générale III de 1:1,80 à 1:2,25.

9. Procédé pour la préparation de silanes contenant de l'urée, de formule générale I, selon la revendication 1, **caractérisé en ce qu'**ensuite on sépare par filtration le produit de réaction et on le lave avec de l'eau et/ou un solvant organique.

10. Procédé pour la préparation de silanes contenant de l'urée, de formule générale I, selon la revendication 9, **caractérisé en ce qu'**on sèche le produit séparé par filtration.
